# EUROPEAN PATENT APPLICATION

(11) **EP 3 517 097 A1**
(43) Date of publication of application: **31.07.2019**
(21) Application number: 17853322.0
(22) Date of filing: 01.09.2017
(51) Int. Cl.: A61K 8/37, A61K 8/92, A61K 8/66, A61K 8/19, A61K 8/29, A61Q 17/04, A61Q 19/00

(54) **FATTY ACID ETHYL ESTER FOR COSMETIC PRODUCT**

(30) Priority: 23.09.2016 KR 20160122416
(71) Applicant: CJ Cheiljedang Corporation, Seoul 04560 (KR)
(72) Inventor: LEE, Yunjeong, Seongnam-si Gyeonggi-do 13595 (KR); KIM, Mijung, Suwon-si Gyeonggi-do 16593 (KR); OH, Han Na, Suwon-si Gyeonggi-do 16509 (KR); PARK, Seung Won, Yongin-si Gyeonggi-do 16894 (KR); LEE, Sang Bum, Seoul 08202 (KR); CHO, Seong Jun, Seoul 04971 (KR)
(74) Representative: Regimbeau
(86) International application number: PCT/KR2017/009624
(87) International publication number: WO 2018/056608

(57) **Abstract**

The present disclosure relates to a solubilizing agent of a UV-blocking agent or a solubilizing agent of a color pigment comprising a C₆₋₁₆ saturated fatty acid ethyl ester, and a cosmetic composition comprising the same.

## Description

### [Technical Field]

The present disclosure relates to a solubilizing agent of an ultraviolet (UV)-blocking agent or color pigment containing a C₆₋₁₆ saturated fatty acid ethyl ester, and a cosmetic composition containing the solubilizing agent.

### [Background Art]

In the formulation of cosmetics, additional ingredients such as a solubilizing agent or dispersant are used as additives so that the active ingredients constituting the cosmetics can be well mixed with a solvent and do not form precipitates with other ingredients. Examples of the solubilizing agent or dispersant generally used at present include polyglycerin fatty acid esters, sorbitan fatty acid esters, silicone surfactants, polyglucosides, ethyl benzoate, C₁₂₋₁₅ alkyl benzo, benzyl benzoate, isoparaffin, methanol, ethanol, propanol, isopropanol, acetone, nonoxynol-12, *etc.* However, these materials are produced in a high-temperature, high-pressure environment or are produced using a chemical catalyst, a solvent, *etc.* For example, 2-phenylethyl benzoate can be prepared by reacting 2-phenylethyl alcohol with benzoic acid using a strong acid catalyst such as methanesulfonic acid at high temperature (180°C or higher) as disclosed in Korean Patent No. 10-1233939. However, when catalysts or solvents produced by a chemical reaction are not completely removed from the final product and remain as a residue, they may cause problems. In particular, the residues of chemicals in cosmetics which are directly applied to the skin can cause serious skin problems. To solve these problems, there is a need for a material which has a function as a raw material for cosmetics and which is produced by an environmentally-friendly method, and which does not cause skin irritation.

The inventors of the present disclosure have conducted studies with regard to a safe and environmentally friendly cosmetic composition, and as a result, have succeeded in preparing a fatty acid ethyl ester suitable for use as a solubilizing agent or dispersant of a UV-blocking agent or color pigment, thereby completing the present disclosure.

### [Disclosure]

### [Technical Problem]

An object of the present disclosure is to provide a solubilizing agent of a UV-blocking agent or color pigment, containing a C₆₋₁₆ saturated fatty acid ethyl ester.

Another object of the present disclosure is to provide a cosmetic composition which contains a solubilizing agent of a UV-blocking agent or color pigment, containing a C₆₋₁₆ saturated fatty acid ethyl ester; and a UV-blocking agent or color pigment.

### [Technical Solution]

The inventors of the present disclosure have conducted studies with regard to a safe and environmentally friendly cosmetic composition, and as a result, have succeeded in preparing a fatty acid ethyl ester suitable for use as a solubilizing agent or dispersant of a UV-blocking agent or color pigment, thereby completing the present disclosure.

### [Advantageous Effects of the Invention]

The C₆₋₁₆ saturated fatty acid ethyl ester of the present disclosure not only has high oxidation stability but also has good miscibility with UV-blocking agents or color pigments, and can therefore be used as a solubilizing agent for UV-blocking agents or coloring pigments. Additionally, fatty acid ethyl esters are present in a liquid state at room temperature and can be widely used for a cosmetic composition of various formulations since they do not deteriorate even when stored for a long period of time.

### [Best Mode for Carrying Out the Invention]

In order to achieve the above objects, an aspect of the present disclosure provides a solubilizing agent of a UV-blocking agent or color pigment, containing a C₆₋₁₆ saturated fatty acid ethyl ester.

Another aspect of the present disclosure provides a cosmetic composition which contains a solubilizing agent of a UV-blocking agent or color pigment, containing a C₆₋₁₆ saturated fatty acid ethyl ester; and a UV-blocking agent or color pigment.

Hereinafter, the present disclosure is explained in detail.

The present disclosure relates to a fatty acid ethyl ester containing saturated fatty acid having a particular carbon number, *i.e.,* a C₆₋₁₆ saturated fatty acid ethyl ester. The fatty acid ethyl ester not only has high oxidation stability but also has excellent miscibility with UV-blocking agents or color pigments. Therefore, when a cosmetic composition is manufactured using the C₆₋₁₆ saturated fatty acid ethyl ester, the desired formulation can be realized by increasing the solubility of various components. Additionally, the fatty acid ethyl ester has excellent miscibility with various components and does not deteriorate even after storage for a long period of time, and thus is suitable for use as a cosmetic ingredient.

The present disclosure provides a solubilizing agent of a UV-blocking agent or color pigment, containing a C₆₋₁₆ saturated fatty acid ethyl ester.

The C₆₋₁₆ saturated fatty acid ethyl ester is prepared via transesterification by contacting a vegetable lipid with lipase. The C₆₋₁₆ saturated fatty acid ethyl ester may also be synthesized by a reaction using a chemical catalyst, but is not limited thereto. More specifically, it can be produced through enzymatic transesterification to meet the intended use for UV-blocking agents or color pigments used in cosmetics.

The C₆₋₁₆ saturated fatty acid ethyl ester may be a caprylic acid ethyl ester, a capric acid ethyl ester, a lauric acid ethyl ester, a myristic acid ethyl ester, a palmitic acid ethyl ester, or a mixture thereof, and more specifically, a caprylic acid ethyl ester, a capric acid ethyl ester, a mixture thereof, or a palmitic acid ethyl ester, but is not limited to.

The vegetable lipid, which can be used for the preparation of the C₆₋₁₆ saturated fatty acid ethyl ester, may be at least one selected from the group consisting of palm oil, palm kernel oil, and coconut oil.

Alternatively, the vegetable lipid may be fractionated to obtain a fat fraction containing 1,3-dipalmitoyl-2-oleoylglycerol (POP) for use as the vegetable lipid. The fractionation may be performed by dry fractionation or solvent fractionation, but is not limited thereto, and any method that can provide a POP-containing oil with a difference in the content of saturated fatty acids and unsaturated fatty acids from the raw materials for the vegetable lipid known in the art may be used without limitation. For example, the fractionation method may be selectively used depending on the characteristics of the raw materials for the vegetable lipid to be used. In the case of solvent fractionation, an organic solvent capable of dissolving the raw materials for the vegetable lipid, such as hexane, acetone, methyl ethyl ketone, ethanol, *etc.,* can be used without limitation. The vegetable lipid to be used in the present disclosure may be a palm mid fraction or an oil, which contains 35 wt% to 70 wt% of 1,3-dipalmitoyl-2-oleoylglycerol (POP) and has an iodine value of 35 to 55.

Specifically, the lipase may be an enzyme having specificity at any of the sn-1,3 positions used in the art, and non-limiting examples of the lipase may include lipases derived from *Rhizopus delemar, RhizoMucor miehei, Aspergillus miger, Rhizopus arrhizus, Rhizopus niveus, Mucor miehei, Mucor javanicus, Rhizopus javenicus, Rhizopus oxyzae,* and *Thermomyces lanuginosus,* and more specifically, lipases derived from *Rhizopus delemar, Mucor miehei,* and *Thermomyces lanuginosus,* but is not limited thereto.

In the present disclosure, the UV-blocking agent may be bis-ethylhexyloxyphenol methoxyphenyl triazine or butyl methoxydibenzoylmethane.

The bis-ethylhexyloxyphenol methoxyphenyl triazine is an oil-soluble compound, which is a broad-spectrum UV absorber that absorbs UVB as well as UVA rays. bis-ethylhexyloxyphenol methoxyphenyl triazine is used as an active ingredient in UV-blocking products designed to protect the skin from the sun after approval as a UV filter in Europe. Meanwhile, in Europe, UV-blocking products are classified as cosmetics, whereas in the U.S.A., UV-blocking products are classified as over-the-counter drugs, and substances approved by the FDA can be used as active ingredients. However, bis-ethylhexyloxyphenol methoxyphenyl triazine has not yet been approved by the U.S. FDA and thus is not used in UV-blocking products, but as a UV absorber in cosmetics and personal beauty care and hygiene products.

The butyl methoxydibenzoylmethane, also known as avobenzone, is an oil-soluble ingredient used in UV-blocking products to absorb the entire spectrum of UVA rays. Butyl methoxydibenzoylmethane has been approved in Europe and by the FDA in America and is now used worldwide. However, butyl methoxydibenzoylmethane is sensitive to properties of solvents and stable to polar protic solvents but unstable in non-polar environments. Butyl methoxydibenzoylmethane may be used along with a photostabilizer because it is decomposed by UV absorption. Non-limiting examples of usable photostabilizers may include octocrylene, 4-methylbenzylidene camphor, bis-ethylhexyloxyphenol methoxyphenyl triazine, methylene bis-benzotriazolyl tetramethylbutylphenol, butyloctyl salicylate, hexadecyl benzoate, butyl octyl benzoate, undecylcrylene dimethicone, terephthalylidene dicamphor sulfonic acid, diethylhexyl 2,6-naphthalate, polyester-8, polysilicon-15, diethylhexyl syringylidene malonate, and ethylhexyl methoxycrylene, or photostability may be improved by using together with cyclodextrin, but the usable photostabilizers are not limited thereto.

In the present disclosure, the color pigment is a material containing a pigment as an active ingredient added to impart a color to a cosmetic composition, and it may be an organic or inorganic material containing a chromophore that can be added to a cosmetic composition to realize a desired color. Specifically, the color pigment may include at least one selected from the group consisting of titanium dioxide, yellow iron oxide, red iron oxide, and black iron oxide, but the color pigment may not be limited thereto and may include color pigments for cosmetics known in the art.

The titanium dioxide is a material widely used as a dye in everything from paints to UV-blocking agents and even foods. The yellow iron oxide is an iron oxide hydroxide in the form of a monohydrate represented by the formula FeO(OH)·H₂O, also indicated as Fe(OH)₃, and also called a hydrated iron oxide. The yellow iron oxide is an FDA-approved material and is used in cosmetics and tattoo inks.

The red iron oxide is a trivalent iron oxide represented by the formula Fe₂O₃, which is contained in "Pigment Brown 6", "Pigment Brown 7", and "Pigment Red 101". The red iron oxide is an FDA-approved material and is used in cosmetics.

The black iron oxide is an iron oxide in which bivalent and trivalent iron coexist, represented by the formula of Fe₃O₄, and is also represented by FeO·Fe₂O₃. The black iron oxide is a black dye known as C.I. pigment black 11.

Additionally, the present disclosure may provide a UV-blocking dispersant or color pigment dispersant containing a C₆₋₁₆ saturated fatty acid ethyl ester.

As used herein, the term "dispersant" may collectively refer to a material capable of uniformly dispersing solid particles in water.

Furthermore, a cosmetic composition containing a solubilizing agent or a cosmetic composition containing the dispersant according to the present disclosure is provided.

Furthermore, a cosmetic composition containing the solubilizing agent of a UV-blocking agent or color pigment containing a C₆₋₁₆ saturated fatty acid ethyl ester; and a UV-blocking agent or color pigment is provided.

The amount of the UV-blocking agent or color pigment with respect to the solubilizing agent may be 10 parts by weight to 120 parts by weight based on 100 parts by weight of the solubilizing agent.

The cosmetic composition may be prepared into a formulation selected from the group consisting of a solution, an ointment for external use, a cream, a foam, a nutrition emollient, a soft emollient, a pack, a soft water, an emulsion, a makeup base, an essence, lip balm, a soap, a liquid cleansing agent, a bath preparation, a sunscreen cream, a sun oil, a suspension, a paste, a gel, a lotion, powders, a surfactant-containing cleansing agent, an oil, a powder foundation, an emulsion foundation, a wax foundation, a patch, and a spray, but the formulation is not limited thereto.

Additionally, the cosmetic composition of the present disclosure may further contain at least one kind of a cosmetically acceptable carrier to be mixed into a common cosmetic composition for the skin, and for example, commonly used ingredients such as a fat content, water, a surfactant, a moisturizer, a lower alcohol, a thickener, a chelating agent, a pigment, a preservative, a perfume, *etc.,* may be appropriately mixed therein, but the additional ingredient is not limited thereto.

Alternatively, the cosmetic composition of the present disclosure may be prepared into functional cosmetics by further containing a component having an antioxidative effect, a wrinkle-improving effect, an anti-aging effect, and/or a UV-blocking effect. For the ingredient having the antioxidative effect, wrinkle-improving effect, anti-aging effect, and/or UV-blocking effect, any active ingredient used in functional cosmetics known in the art may be used without limitation.

The solubilizing agent or dispersant containing a C₆₋₁₆ saturated fatty acid ethyl ester of the present disclosure has excellent miscibility when prepared into a cosmetic product by adding other raw materials thereto for the above purpose. Additionally, since the solubilizing agent or dispersant of the present disclosure has the same or higher oxidative stability and physical properties compared to those of the materials which have been conventionally used in cosmetics for the same purpose, it is possible to formulate the solubilizing agent or dispersant of the present disclosure into cosmetics.

More specifically, the present disclosure may provide a cosmetic composition which contains at least one kind of a fatty acid ester selected from the group consisting of caprylic acid ethyl ester, capric acid ethyl ester, and palmitic acid ethyl ester; and a UV-blocking agent or color pigment.

The amount of the UV-blocking agent or color pigment relative to the fatty acid ester may each be 10 parts by weight to 120 parts by weight, 15 parts by weight to 110 parts by weight, 20 parts by weight to 100 parts by weight, 25 parts by weight to 90 parts by weight, 30 parts by weight to 80 parts by weight, 35 parts by weight to 70 parts by weight, or 40 parts by weight to 60 parts by weight, based on 100 parts by weight of the fatty acid ester. For more specific details on the UV-blocking agent or color pigment, one may refer to those described above.

### [Mode for Carrying out the Invention]

Hereinafter, the present disclosure will be described in detail through specific examples. However, these examples are intended to more particularly illustrate the present disclosure and the scope of the present disclosure is not limited by these examples.

### Preparation Example 1: Preparation of fatty acid ethyl ester by enzymatic transesterification

Triglycerides, a palm mid fraction (hereinafter, PMF) having a high palmitate content, was mixed with stearic acid ethyl ester at a 1:2 molar ratio to a final volume of 1 L. In particular, the PMF contained 55% POP and had an iodine value of 40. The specific details of the preparation method are described below:
Palm oil (1 kg) was completely dissolved at 60°C and sealed by mixing with acetone (10 kg), and the mixture was stirred to completely dissolve the oil in acetone. The mixture was crystallized by stirring at 30 rpm for 3 hours at 0°C, and the resultant was filtered under reduced pressure and separated into palm stearin (solid phase) into palm olein (liquid phase). In particular, the yield of palm olein was 60% or higher and the palm olein exhibited an iodine value of 60 or less. The palm stearin obtained by the fractionation, in which the acetone was not removed, was completely dissolved at 40°C. Then, acetone was further added thereto and the mixture was crystallized by stirring at 30°C at 30 rpm. The resultant was filtered under reduced pressure and separated into a crystallized fraction and PMF. In particular, the yield of PMF was 30% or higher, and the PMF contained 55% POP and had an iodine value of 40.

The mixture was mixed with a double jacketed 2 L reactor together with 50 g of a position-specific lipase (from *Mucor miehei*) enzyme and allowed to react at 50°C for 6 hours. Palmitic acid ethyl ester was formed by the exchange of palmitic acid, which was bound to PMF by lipase, and stearic acid ethyl ester, which was used as a raw material, during the reaction. Distillation was performed to separate triglycerides from the mixture after completion of the reaction. The fatty acid ethyl ester was obtained by distillation of triglycerides under the conditions of 2 mbar vacuum, a column bottom temperature of 235°C, and a reflux ratio of 1 in a 4-stage distillation tower. Further distillation was performed to improve the purity of palmitic acid ethyl ester. Specifically, palmitic acid ethyl ester having a purity of 98% or higher (Example 2) was obtained by distillation of under the conditions of 2 mbar vacuum, a column bottom temperature of 235°C, and a reflux ratio of 3 in a 22-stage distillation tower.

To compare stability and solubility or dispersion effects of fatty acid ethyl esters, a caprylic acid/capric acid ethyl ester (Example 1), a stearic acid ethyl ester (Comparative Example 1), and an oleic acid ethyl ester (Comparative Example 2) were prepared according to a method similar to that described above. For Comparative Examples 3 and 4, a fatty acid ethyl ester complex and C₁₂₋₁₅ alkyl benzoate were used. The main ingredients of these samples are shown in Table 1 below.

**[Table 1]**

| | |
|---|---|
| Example 1 | Caprylic acid/Capric acid ethyl ester |
| Example 2 | Palmitic acid ethyl ester |
| Comparative Example 1 | Stearic acid ethyl ester |
| Comparative Example 2 | Oleic acid ethyl ester |
| Comparative Example 3 | Fatty acid ethyl ester complex (unsaturated fatty acid ethyl ester 80%) |
| Comparative Example 4 | C₁₂₋₁₅ alkyl benzoate |

### Experimental Example 1: Comparative evaluation of solubility of organic UV-blocking agents

Organic UV-blocking agents (bis-ethylhexyloxyphenol methoxyphenyl triazine and butyl methoxybenzoylmethane) were dissolved with heat treatment at 70°C at a concentration of 25% for 10 minutes using the fatty acid ethyl esters of Examples 1 and 2 and Comparative Examples 1 to 4 as a solubilizing agent, respectively. For the evaluation of their stability, the dissolved UV-blocking agents were left to stand for 1 week in a 25°C thermostat, and the presence or absence of precipitation on Day 1, Day 3, and Day 7 was visually confirmed. The results for each UV-blocking agent are shown in Tables 2 and 3.

**[Table 2]**

| Presence of precipitation | Ex. 1 | Ex. 2 | Comp. Ex. 1 | Comp. Ex. 2 | Comp. Ex. 3 | Comp. Ex. 4 |
|---|---|---|---|---|---|---|
| Day 1 | Precipitation (X) | Precipitation (X) | Unable to determine | Precipitation (X) | Precipitation (X) | Precipitation (X) |
| Day 3 | Precipitation (X) | Precipitation (X) | Unable to determine | Precipitation (X) | Precipitation (O) | Precipitation (X) |
| Day 7 | Precipitation (X) | Precipitation (X) | Unable to determine | Precipitation (X) | Precipitation (O) | Precipitation (X) |

Table 2 above shows the results of stability evaluated when bis-ethylhexyloxyphenol methoxyphenyl triazine was dissolved in each of the solubilizing agents at a concentration of 25%.

**[Table 3]**

| Presence of precipitation | Ex. 1 | Ex. 2 | Comp. Ex. 1 | Comp. Ex. 2 | Comp. Ex. 3 | Comp. Ex. 4 |
|---|---|---|---|---|---|---|
| Day 1 | Precipitation (X) | Precipitation (X) | Precipitation (O) | Precipitation (X) | Precipitation (X) | Precipitation (X) |
| Day 3 | Precipitation (X) | Precipitation (X) | Precipitation (O) | Precipitation (X) | Precipitation (O) | Precipitation (X) |
| Day 7 | Precipitation (X) | Precipitation (X) | Precipitation (O) | Precipitation (X) | Precipitation (O) | Precipitation (O) |

Table 3 above shows the results of stability evaluated when butyl methoxybenzoylmethane was dissolved in each of the solubilizing agents at a concentration of 25%.

As shown in Tables 2 and 3, when the fatty acid ethyl esters of Examples 1 and 2 were used as a solubilizing agent, respectively, organic UV-blocking agents were stably dissolved for one week. Meanwhile, when a large amount of an unsaturated fatty acid ethyl ester, contained in Comparative Example 3, or an alkyl benzoate, contained in Comparative Example 4, was used as a solubilizing agent, the organic UV-blocking agents were precipitated after Day 3 or Day 7. Meanwhile, since the C₁₈ stearic acid ethyl ester of Comparative Example 1 itself is present in a solid state at 25°C, it was not possible to determine whether the active ingredient was precipitated. That is, it was confirmed that the fatty acid ethyl esters of Example 1 and Example 2 were very suitable as a solubilizing agent for organic UV-blocking agents.

### Experimental Example 2: Oxidation stability of fatty acid ethyl esters

The oxidation stability of the fatty acid ethyl esters prepared by transesterification with the enzyme according to Preparation Example 1 was confirmed and the results are shown in Table 4 below. The oxidation stability was analyzed according to the AOCS Cd 12b-92 test method.

The oxidation stability test method is a test method for measuring the oxidation stability of oils.

**[Table 4]**

| | Oxidation stability (hours) |
|---|---|
| Ex. 1 | 63 |
| Ex. 2 | 67 |
| Comp. Ex. 1 | 68 |
| Comp. Ex. 2 | 7 |
| Comp. Ex. 3 | 11 |

As shown in Table 4, it was confirmed that the saturated fatty acid ethyl esters (Example 1, Example 2, and Comparative Example 1) showed excellent oxidation stability compared to the unsaturated fatty acid ethyl esters (Comparative Example 2 and Comparative Example 3). In the case of Comparative Example 3, although the saturated fatty acid ethyl ester contains saturated fatty acid to some extent, it is thought that the oxidation stability was significantly lowered due to the high content of unsaturated fatty acid.

### Experimental Example 3: Evaluation of formulations of UV-blocking agents

UV-blocking agents were prepared using the fatty acid ethyl esters of Examples 1 and 2 and Comparative Examples 3 and 4, and the constituent ingredients and amount of each composition are shown in Tables 5 and 6 below. Referring to Experimental Examples 1 and 2 above, the fatty acid ethyl esters of Comparative Examples 1 and 2 were determined to be unacceptable as raw materials for cosmetics, and thus, these were excluded in Experimental Example 3.

**[Table 5]**

| Name of Raw Materials (Korean) | wt% | wt% | wt% | wt% |
|---|---|---|---|---|
| Distilled water | to 100 | to 100 | to 100 | to 100 |
| Acrylate/C10-30 Alkyl acrylate crosspolymer | 0.2 | 0.2 | 0.2 | 0.2 |
| Butylene glycol | 5 | 5 | 5 | 5 |
| Disodium EDTA | Adequate | Adequate | Adequate | Adequate |
| Ex. 1 | 10 | - | - | - |
| Ex. 2 | - | 10 | - | - |
| Comp. Ex. 3 | - | - | 10 | - |
| Comp. Ex. 4 | - | - | - | 10 |
| Bis-ethylhexyloxyphenol methoxyphenyl triazine | 5 | 5 | 5 | 5 |
| Octocrylene | 3 | 3 | 3 | 3 |
| Ethylhexyl methoxycinnamate | 7 | 7 | 7 | 7 |
| Ethylhexyl salicylate | 5 | 5 | 5 | 5 |
| Stearic acid | 1.5 | 1.5 | 1.5 | 1.5 |
| Cetearyl alcohol | 2 | 2 | 2 | 2 |
| Behenyl alcohol | 0.5 | 0.5 | 0.5 | 0.5 |
| Cyclopentasiloxane/ Cyclohexasiloxane | 5 | 5 | 5 | 5 |
| Triethanolamine | 0.2 | 0.2 | 0.2 | 0.2 |
| Preservative, Fragrance | Adequate | Adequate | Adequate | Adequate |

**[Table 6]**

| Name of Raw Materials (Korean) | wt% | wt% | wt% | wt% |
|---|---|---|---|---|
| Distilled water | to 100 | to 100 | to 100 | to 100 |
| Acrylate/C10-30 Alkyl acrylate crosspolymer | 0.2 | 0.2 | 0.2 | 0.2 |
| Butylene glycol | 5 | 5 | 5 | 5 |
| Disodium EDTA | Adequate | Adequate | Adequate | Adequate |
| Ex. 1 | 10 | - | - | - |
| Ex. 2 | - | 10 | - | - |
| Comp. Ex. 3 | - | - | 10 | - |
| Comp. Ex. 4 | - | - | - | 10 |
| Butyl methoxydibenzoylmethane | 5 | 5 | 5 | 5 |
| Octocrylene | 3 | 3 | 3 | 3 |
| Ethylhexyl methoxycinnamate | 7 | 7 | 7 | 7 |
| Ethylhexyl salicylate | 5 | 5 | 5 | 5 |
| Stearic acid | 1.5 | 1.5 | 1.5 | 1.5 |
| Cetearyl alcohol | 2 | 2 | 2 | 2 |
| Behenyl alcohol | 0.5 | 0.5 | 0.5 | 0.5 |
| Cyclopentasiloxane/ Cyclohexasiloxane | 5 | 5 | 5 | 5 |
| Triethanolamine | 0.2 | 0.2 | 0.2 | 0.2 |
| Preservative, Fragrance | Adequate | Adequate | Adequate | Adequate |

In order to evaluate the stability of each of the compositions, these compositions were left at room temperature (about 25°C) and 45°C for 30 days, respectively, and comparative evaluation of their properties and stability were visually confirmed, and the results are shown in Table 7 below. Those cases where no change was observed were indicated by X.

**[Table 7]**

| | Ex. 1 | Ex. 2 | Comp. Ex. 3 | Comp. Ex. 4 |
|---|---|---|---|---|
| 25°C | X | X | ○ | X |
| 45°C | X | X | ○ | X |

As shown in Table 7, it was confirmed that the UV-blocking agents containing the fatty acid ethyl esters of Examples 1 and 2 remained stable without any physical or chemical change such as discoloration, change of odors, phase separation, *etc.,* even when exposed to room temperature or high temperature for 30 days. This suggests that the addition of the fatty acid ethyl ester of the present disclosure as an additive to promote the dissolution of the active ingredients will not cause any change in the quality of the formulation as the fatty acid ethyl ester is well mixed with other cosmetic compositions and it is also suitable for use as a cosmetic composition because it has excellent stability even after long-term storage at high temperature.

### Experimental Example 4: Evaluation of foundation formulations

Foundations were prepared based on the ingredients and the amounts shown in Table 8 below.

**[Table 8]**

| | wt% | wt% | wt% | wt% |
|---|---|---|---|---|
| Ex. 1 | 10 | - | - | - |
| Ex. 2 | - | 10 | - | - |
| Comp. Ex. 3 | - | - | 10 | - |
| Comp. Ex. 4 | - | - | - | 10 |
| Ethylhexyl methoxycinnamate | 7 | 7 | 7 | 7 |
| Bis-ethylhexyloxyphenol methoxyphenyl triazine | 4 | 4 | 4 | 4 |
| Cyclopentasiloxane/ Cyclohexasiloxane | 10 | 10 | 10 | 10 |
| Cetyl PEG/PPG-10/1 dimethicone | 1 | 2 | 2 | 2 |
| PEG-9 polydimethylsiloxyethyl dimethicone | 2 | 2 | 2 | 2 |
| Sorbitan sesquioleate | 1.5 | 1.5 | 1.5 | 1.5 |
| Titanium dioxide | 10 | 10 | 10 | 10 |
| Disteardimonium hectorite | 1 | 1 | 1 | 1 |
| Butylene glycol | 5 | 5 | 5 | 5 |
| Sodium chloride | 1 | 1 | 1 | 1 |
| Yellow iron oxide | Adequate | Adequate | Adequate | Adequate |
| Red iron oxide | Adequate | Adequate | Adequate | Adequate |
| Black iron oxide | Adequate | Adequate | Adequate | Adequate |
| Disodium EDTA | Adequate | Adequate | Adequate | Adequate |
| Preservative, Fragrance | Adequate | Adequate | Adequate | Adequate |
| Distilled water | to 100 | to 100 | to 100 | to 100 |

A group of panelists consisting of 30 women and men aged 20 to 40 years were allowed to use foundations prepared according to the compositions shown above for one week, and the results were compared and evaluated. Evaluation items consisted of spreadability, adhesion, durability, applicability, and color expression.

The evaluation criteria were 1 (very unsatisfied) to 10 (very satisfied), and the sensory evaluation results according to each evaluation item were collected and shown in Table 9 below.

**[Table 9]**

| | Ex. 1 | Ex. 2 | Comp. Ex. 3 | Comp. Ex. 4 |
|---|---|---|---|---|
| Spreadability | 9.5 | 9.8 | 6.2 | 7.8 |
| Adhesion | 9.0 | 9.5 | 5.8 | 7.6 |
| Durability | 9.2 | 9.7 | 6.3 | 7.1 |
| Applicability | 9.4 | 9.6 | 5.5 | 6.9 |
| Color Expression | 9.3 | 9.8 | 5.9 | 7.3 |

As shown in Table 9 above, those foundations which contained fatty acid ethyl esters of Examples 1 and 2 according to the present disclosure were evaluated to be excellent in all of the evaluation items compared to the foundations of Comparative Examples, and in particular, these foundations were shown to have excellent color expression.

## Claims

1. A solubilizing agent of a UV-blocking agent or color pigment, comprising a C₆₋₁₆ saturated fatty acid ethyl ester.

2. The solubilizing agent according to claim 1, wherein the C₆₋₁₆ saturated fatty acid ethyl ester is prepared via transesterification by contacting a vegetable lipid with lipase.

3. The solubilizing agent according to claim 2, wherein the C₆₋₁₆ saturated fatty acid ethyl ester is a caprylic acid ethyl ester, a capric acid ethyl ester, a lauric acid ethyl ester, a myristic acid ethyl ester, a palmitic acid ethyl ester, or a mixture thereof.

4. The solubilizing agent according to claim 2, wherein the vegetable lipid is at least one selected from the group consisting of palm oil, palm kernel oil, and coconut oil.

5. The solubilizing agent according to claim 2, wherein the lipase is a lipase derived from *Mucor miehei* or *Thermomyces lanuginosus.*

6. The solubilizing agent according to claim 1, wherein the UV-blocking agent is bis-ethylhexyloxyphenol methoxyphenyl triazine or butyl methoxydibenzoylmethane.

7. The solubilizing agent according to claim 1, wherein the color pigment is at least one selected from the group consisting of titanium dioxide, yellow iron oxide, red iron oxide, and black iron oxide.

8. A cosmetic composition comprising the solubilizing agent according to claim 1; and a UV-blocking agent or color pigment.

9. The cosmetic composition according to claim 8, wherein the UV-blocking agent or color pigment is comprised in an amount of 10 parts by weight to 120 parts by weight based on 100 parts by weight of the solubilizing agent.
